# EUROPÄISCHE PATENTSCHRIFT

(11) **EP 3 445 068 B1**
(45) Veröffentlichungstag und Bekanntmachung des Hinweises auf die Patenterteilung: **22.01.2020**
(21) Anmeldenummer: 18186744.1
(22) Anmeldetag: 01.08.2018
(51) Int. Cl.: H04R 25/00, H04R 1/10, H04R 5/033

(54) **VERFAHREN ZUM BETRIEB EINES HÖRGERÄTS UND HÖRGERÄT**
HEARING AID AND METHOD FOR OPERATING A HEARING AID
PROCÉDÉ DE FONCTIONNEMENT D'UN APPAREIL DE CORRECTION ADDITIVE ET APPAREIL DE CORRECTION ADDITIVE

(30) Priorität: 14.08.2017 DE 102017214163
(43) Veröffentlichungstag der Anmeldung: 20.02.2019
(73) Patentinhaber: Sivantos Pte. Ltd., Singapore 539775 (SG)
(72) Erfinder: HANNEMANN, Ronny, 91054 Buckenhof (DE); STRAUSS, Daniel J., 66129 Saarbrücken (DE); CORONA-STRAUSS, Farah I., 66129 Saarbrücken (DE)
(74) Vertreter: FDST Patentanwälte

(56) Entgegenhaltungen:
- EP-A2- 2 200 347
- KR-A- 20160 129 752
- US-A1- 2010 160 714
- US-A1- 2012 177 233
- US-A1- 2014 098 981
- US-A1- 2016 119 726

## Beschreibung

Die Erfindung betrifft ein Verfahren zum Betrieb eines Hörgeräts sowie ein entsprechendes Hörgerät.

Ein Hörgerät dient allgemein der Wiedergabe eines Schallsignals in Richtung eines Ohrs eines Anwenders. Das Hörgerät wird hierzu im oder am Ohr getragen und weist einen Hörer auf, über welchen eine Schallausgabe erfolgt. Im Speziellen dient ein Hörgerät der Versorgung eines hörgeschädigten Anwenders. Ein solches Hörgerät weist üblicherweise eine Anzahl an Mikrofonen auf, zur Aufnahme von Schallsignalen aus der Umgebung, und eine Signalverarbeitungseinheit, welche die aufgenommenen Schallsignale geeignet modifiziert, insbesondere verstärkt, und dann an den Hörer zur Ausgabe weiterleitet.

Der Nutzen eines solchen Hörgeräts für den Anwender hängt wesentlich von der Fähigkeit des Hörgeräts ab, die Schallsignale derart auszugeben, dass diese den Bedürfnissen des Anwenders in einer konkreten Situation möglichst optimal entsprechen. Dies geschieht über die Einstellung einer Anzahl an Betriebsparametern des Hörgeräts, welche dann dessen Verhalten im Betrieb definieren.

Zur Wahrnehmung von Schallsignalen ist ganz allgemein ein gewisses Maß an Anstrengung erforderlich. Diese Anstrengung wird auch als Höranstrengung (Englisch: listening effort) bezeichnet. Die Höranstrengung ist dabei größer z.B. in Umgebungen, welche eine Vielzahl konkurrierender Schallquellen enthalten und geringer z.B. in ruhigen Umgebungen. Eine Definition für die Höranstrengung ist unter Anderem gegeben in Bernarding et al., "Neurodynamic evaluation of hearing aid features using EEG correlates of listening effort", Cognitive Neurodynamics, 2017, DOI 10.1007/s11571-017-9425-5. Darin wird die Höranstrengung definiert als eine mentale Anstrengung des Anwenders, um Schallsignale, d.h. auditorische Reize zu verarbeiten, besonders in schwierigen Umgebungen, d.h. in Umgebungen mit Störeinflüssen oder mit einer Vielzahl von Schallsignalen. Es handelt sich also nicht um eine reflexartige Handlung, sondern um eine vom Anwender aktiv gewollte und vorgenommene Anstrengung. Daraus resultiert eine andauernde und oszillierende Aktivität, genauer EEG-Aktivität.

Für hörgeschädigte Menschen ist im Vergleich zu normalhörenden Menschen die Höranstrengung in derselben Situation zuweilen deutlich größer, sodass schneller eine Ermüdung eintritt. Unter Umständen versucht der hörgeschädigte Mensch sogar, Situationen mit hoher Höranstrengung zu umgehen oder zu vermeiden, was zu einer verringerten sozialen Aktivität und letztendlich zu einer verminderten Lebensqualität führen kann. Daher ist es erstrebenswert, hörgeschädigte oder auch lediglich hörbeeinträchtigte Menschen mit einem Hörgerät derart zu versorgen, dass die Höranstrengung minimiert wird.

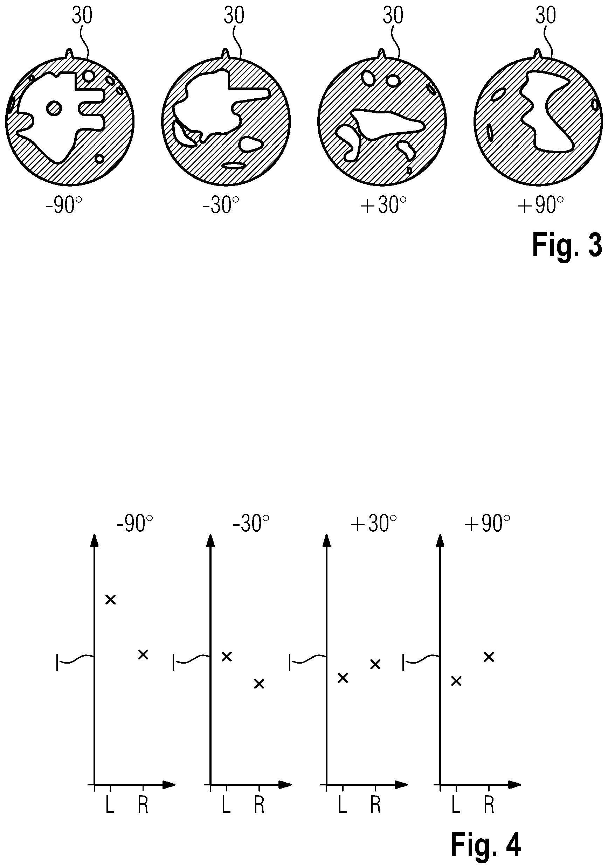

In der EP 2 357 851 A1 ist ein Verfahren beschrieben, bei welchem zunächst die Höranstrengung in bestimmten Trainingssituationen mittels eines Elektroenzephalogramms, kurz EEG, ermittelt wird. Durch eine Anpassung der Hörgeräteparameter wird dann versucht, die Höranstrengung zu minimieren. Dabei wird aus dem EEG ein Wert abgeleitet, welcher dann als Maß für die Höranstrengung dient. Auch in Bernarding et al. (a.a.O.) wird ein Wert für die Höranstrengung aus einem EEG abgeleitet. Dort wurden Hörgeräte in verschiedenen Betriebsmodi betrieben und für diese Betriebsmodi jeweils die Höranstrengung ermittelt, vgl. dort S.3 Abschnitt "Hearing aid fitting" und Fig.3 auf S.8.

In der EP 2 200 347 A2 wird ein Verfahren zum Betrieb eines Hörinstruments beschrieben. Dabei wird eine Kapazität des Arbeitsgedächtnisses eines Nutzers abgeschätzt. Basierend darauf wird eine kognitive Belastung des Nutzers abgeschätzt. Schließlich wird die Verarbeitung eines Eingangssignals abhängig von der geschätzten kognitiven Belastung angepasst.

Vor diesem Hintergrund ist es eine Aufgabe der Erfindung ein verbessertes Verfahren zum Betrieb eines Hörgeräts anzugeben sowie ein entsprechendes Hörgerät. Dabei soll die Höranstrengung eines Anwenders des Hörgeräts möglichst stark reduziert werden.

Die Aufgabe wird erfindungsgemäß gelöst durch ein Verfahren mit den Merkmalen gemäß Anspruch 1 sowie durch ein Hörgerät mit den Merkmalen gemäß Anspruch 15. Vorteilhafte Ausgestaltungen, Weiterbildungen und Varianten sind Gegenstand der Unteransprüche. Dabei gelten die Ausführungen im Zusammenhang mit dem Verfahren sinngemäß auch für das Hörgerät und umgekehrt.

Das Verfahren dient zum Betrieb eines Hörgeräts. Das Hörgerät ist zur Anwendung durch einen Anwender ausgebildet. Im Rahmen des Verfahren wird ein neuronales Signal des Anwenders gemessen und daraus eine Höranstrengung des Anwenders ermittelt wird. Dabei werden ausschließlich aus dem neuronalen Signal sowohl eine Intensität der Höranstrengung als auch eine Hörrichtung ermittelt. Die Intensität und die Hörrichtung bilden einen Hörvektor, wobei die Intensität einer Länge des Hörvektors entspricht und die Hörrichtung einer Orientierung des Hörvektors. Der Hörvektor wird auch als Höranstrengungsvektor bezeichnet. In Abhängigkeit der Hörrichtung im Speziellen und des Hörvektors im Allgemeinen wird dann ein Betriebsmodus des Hörgeräts angepasst oder eingestellt, zur Reduzierung der Intensität der Höranstrengung. Es erfolgt also eine Anpassung des aktuellen Betriebsmodus oder ein Umschalten in einen anderen Betriebsmodus in Abhängigkeit der Höranstrengung.

Ein wesentlicher Vorteil der Erfindung besteht insbesondere darin, dass nicht lediglich die Intensität der Höranstrengung bestimmt wird, sondern auch die Hörrichtung, d.h. diejenige Richtung, in welche der Anwender hören will. Diese Hörrichtung wird verwendet, um den Betrieb des Hörgeräts zu optimieren. In dem neuronalen Signal ist nämlich die Intention des Anwenders enthalten, sodass aus dem Hörvektor der Willen des Anwenders extrahiert werden kann und auch wird. Dieser Willen äußert sich einerseits in der Intensität, welche ein Interesse des Anwenders angibt, etwas Bestimmtes zu hören, und andererseits auch in der Hörrichtung, welche angibt, wohin das Interesse des Anwenders gerichtet ist. Der Willen oder genauer der Hörwillen oder das Hörinteresse wird also durch Bestimmung der Hörrichtung präziser festgestellt. Die Kenntnis der Hörrichtung ermöglicht dann eine deutlich zielgerichtetere und bedarfsgerechtere Einstellung des Hörgeräts. Zu einer gegebenen Situation, in welcher sich der Anwender befindet, wird dann ein optimaler Betriebsmodus gefunden, sodass die Höranstrengung, genauer gesagt deren Intensität, vorteilhaft reduziert wird. Dies entlastet den Anwender und führt insgesamt zu einer Erhöhung der Lebensqualität.

Ein zentrales Konzept ist insbesondere, die Höranstrengung nicht wie in der EP 2 357 851 A1 oder wie in Bernarding et al. (a.a.O.) als einen reinen Skalar zu ermitteln, welcher lediglich eine Intensität der Höranstrengung angibt, sondern im Gegensatz dazu die Höranstrengung als ein vektorielles Objekt darzustellen, nämlich als einen Vektor, welcher zusätzlich zu der Information der Intensität der Höranstrengung auch eine Information bezüglich der Richtung der Höranstrengung enthält. Dieser Vektor ist dann ein Hörvektor, welcher angibt, in welcher Richtung und mit welcher Anstrengung der Anwender des Hörgeräts hört. Während die Länge des Hörvektors ein Maß für die Anstrengung ist, mit welcher der Anwender versucht zu hören, ist die Orientierung des Hörvektors ein Maß für die Richtung, in welcher der Anwender hören will. Dabei wird der Hörvektor insbesondere ausschließlich, d.h. alleinig aus dem neuronalen Signal abgeleitet und gerade nicht aus einem bezüglich des Anwenders externen Signal, wie beispielsweise einem Schallsignal.

Vorliegend wird durch die Messung des neuronalen Signals gezielt insbesondere eine andauernde oder oszillierende Aktivität (Englisch: oscillatory activity) des Gehirns des Anwenders gemessen. Das neuronale Signal ist demnach gerade kein Resultat eines spezifischen Stimulus und kein ereigniskorreliertes Signal oder auch Potential (Englisch: event related potential). Ein solcher spezifischer Stimulus erzeugt nämlich gerade kein neuronales Signal, welchem sich eine Hörintention entnehmen lässt, sondern lediglich eine reflexartige Reaktion auf einen speziellen Reiz, d.h. ein ereigniskorreliertes Signal. Eine Verwendung ereigniskorrelierter Signale als Resultat spezifischer Stimuli ist beispielsweise beschrieben in Hanson, Odame "Towards a Brain-Machine System for Auditory Scene Analysis", 2015, Wearable Electronic Sensors, S.299ff. DOI: 10.1007/978-3-319-18191-2_13. Ein Signal hängt dann mit einem bestimmten Stimulus zusammen, dagegen wird im Rahmen der vorliegenden Anmeldung die andauernde oder oszillierende Aktivität, d.h. ein andauerndes oder oszillierendes Potential gemessen, welches insbesondere nicht durch spezifische Stimuli erzeugt wird, sondern eine konkrete Hörintention des Anwenders enthält.

Vorliegend werden zudem sowohl die Intensität der Höranstrengung als auch eine Hörrichtung, d.h. zusammenfassend die Höranstrengung, insbesondere ausschließlich aus dem neuronalen Signal bestimmt. Zusätzliche Messungen insbesondere externer Signale sind vorteilhaft nicht notwendig und werden daher zweckmäßigerweise auch nicht zur Bestimmung der Höranstrengung durchgeführt. Vor Allem wird auf eine zusätzliche Mikrofonanalyse zwecks Verarbeitung des neuronalen Signals verzichtet. Der Gegenstand der vorliegenden Anmeldung steht damit im Gegensatz zu dem z.B. in O'Sullivan et al. "Neural decoding of attentional selection in multi-speaker environments without access to clean sources", 2017, Journal of Neural Engineering, DOI: 10.1088/1741-2552/aa7ab4 beschriebenen Verfahren, bei welchem eine Mikrofonanalyse durchgeführt wird, um ein gemessenes EEG-Signal auszuwerten. Dabei wird zur Lösung des Cocktail-Party-Problems die Kenntnis verwendet, dass das EEG-Signal der Einhüllenden der verfolgten Schallquelle folgt, was jedoch nicht einer Ermittlung einer Hörintention entspricht, also auch nicht einer Bestimmung der Höranstrengung. Die Höranstrengung ist insbesondere eine externe Höranstrengung (Englisch: external/perceptual listening effort), d.h. eine Höranstrengung, welche auf externe Objekte gerichtet ist, z.B. konkrete Schallquellen in der Umgebung, im Gegensatz zu einer internen Höranstrengung, welche auf interne Objekte gerichtet ist, wie z.B. die Interpretation oder das Verständnis bestimmter Ausdrücke oder Sprachen. Eine Definition von externer und interner Höranstrengung ist gegeben in Strauss and Francis "Toward a Taxonomic Model of Attention in Effortful Listening", Cognitive, Affective & Behavioral Neuroscience, 2017, DOI 10.3758/s13415-017-0513-0. Dort wird die Höranstrengung als ein Vektor im zweidimensionalen Raum der internen und externen Höranstrengung dargestellt, d.h. die Orientierung des dortigen Vektors gibt ein Verhältnis der internen zur externen Höranstrengung an, wohingegen die Hörrichtung unbeachtet bleibt. Alternativ oder zusätzlich zur externen Höranstrengung wird auch die interne Höranstrengung ermittelt und zur Anpassung oder Einstellung des Betriebsmodus gewinnbringend hinzugezogen. Im Rahmen des Verfahrens wird versucht, das Hörgerät derart einzustellen, dass die Intensität der Höranstrengung reduziert wird. Hierzu wird in Abhängigkeit des Hörvektors der Betriebsmodus eingestellt oder angepasst. Mit anderen Worten: die ermittelte Hörrichtung wird als eine Zusatzinformation für eine Reduzierung der Höranstrengung verwendet. Jeder Betriebsmodus ist definiert durch konkrete Werte für eine Anzahl an Betriebsparametern des Hörgeräts. Solche Betriebsparameter sind z.B. ein Verstärkungsfaktor, ein Kompressionsfaktor, eine Filterbandbreite oder Ähnliches. Die Reduzierung der Höranstrengung, d.h. die Reduzierung der Intensität, erfolgt durch eine Anpassung der Betriebsparameter des Hörgeräts. Die Anpassung erfolgt dabei entweder im Rahmen eines bestimmten Betriebsmodus, welcher dann angepasst wird, oder die Betriebsparameter werden derart angepasst, dass sogar ein anderer Betriebsmodus eingestellt wird. Durch die zusätzliche Kenntnis der Hörrichtung werden diese Betriebsparameter nun deutlich zielgerichteter eingestellt. Dabei wird gezielt die Hörrichtung mit in das Reduzierungskonzept einbezogen, anstatt allgemein zu versuchen, lediglich die Intensität der Höranstrengung zu reduzieren. Mit der Orientierung enthält der Hörvektor ein Maß für die Intention, genauer die Hörintention des Anwenders. In dem Hörvektor ist also die Hörintention des Anwenders kodiert. Die Kenntnis der gewollten Hörrichtung ermöglicht dann eine Reduzierung des Parameterraums, in welchem versucht wird, durch geeignete Wahl der Betriebsparameter die Intensität der Höranstrengung zu minimieren.

Der Betriebsmodus wird vorzugsweise angepasst, indem eine Anzahl an Betriebsparametern des Hörgeräts mittels einer Regelung (Englisch: closed-loop control) angepasst werden, wobei die Betriebsparameter als Stellgrößen verwendet werden und wobei die Intensität der Höranstrengung als Regelgröße verwendet wird. Zweck der Regelung ist eine Minimierung der Intensität der Höranstrengung. Die Betriebsparameter werden also solange angepasst, bis die Intensität einen Minimalwert annimmt. Die Regelung ist geeigneterweise ein Teil einer Steuereinheit des Hörgeräts. Alternativ ist aber auch eine Auslagerung der Regelung zweckmäßig. Die Regelung erfolgt dann auf einem externen Gerät, wodurch Rechenleistung des Hörgeräts gespart wird. Die Regelung berücksichtigt explizit die Hörrichtung, d.h. die Kenntnis der Hörrichtung fließt in die Regelung mit ein. Die Hörrichtung ist dabei insbesondere keine Regelgröße und auch keine Stellgröße, da die Hörrichtung als Hörintention des Anwenders nicht durch das Hörgerät einstellbar ist. Die Hörrichtung dient aber vorteilhaft als Zielgröße für die Einstellung oder Anpassung der Betriebsparameter, nämlich vorzugsweise derart, dass dadurch ein Hören in Hörrichtung vereinfacht wird. Durch die Berücksichtigung der Hörrichtung ist die Regelung insgesamt effektiver, denn mit der Hörrichtung ist eine zusätzliche Randbedingung oder Zielbedingung vorhanden, welche eine optimale Einstellung der Betriebsparameter vereinfacht.

Die Kenntnis der intendierten Hörrichtung des Anwenders eignet sich ganz besonders zur Anpassung des Betriebsmodus hinsichtlich einer Richtcharakteristik des Hörgeräts. In einer bevorzugten Ausgestaltung ist der Betriebsmodus dann ein Richtungshörbetrieb, bei welchem Schallsignale aus einer Vorzugsrichtung relativ zu Schallsignalen aus anderen Richtungen verstärkt ausgegeben werden. Der Richtungshörbetrieb wird nun angepasst, indem die Hörrichtung als die Vorzugsrichtung eingestellt wird. Ohne Kenntnis der Hörrichtung müsste diese aufgrund anderer Informationen geschätzt werden, z.B. durch eine Mikrofonanalyse oder durch Beschleunigungssensoren oder Ähnliches. Solche Methoden basieren jedoch prinzipbedingt ausschließlich auf äußeren Informationen, es verbleibt also immer eine Unsicherheit, ob die eingestellt Vorzugsrichtung tatsächlich die vom Anwender gewollte Richtung ist. Demgegenüber ist im Hörvektor prinzipbedingt die Hörintention des Anwenders enthalten. Durch die Messung und Auswertung des neuronalen Signals wird direkt die tatsächlich gewollte Hörrichtung ermittelt, denn die neuronalen Signale ergeben sich gerade in Abhängigkeit der vom Anwender gewollten Hörrichtung. Die Auswahl der Vorzugsrichtung beim Richtungshören ist somit deutlich weniger fehleranfällig.

Zweckmäßigerweise wird die oben beschriebene Anpassung des Richtungshörbetriebs mit der weiter oben beschriebenen Regelung kombiniert. Die Hörrichtung ist dann geeigneterweise eine Führungsgröße in einer Steuerung oder Regelung für einen der Betriebsparameter, insbesondere der Vorzugsrichtung beim Richtungshörbetrieb. Die Intensität der Höranstrengung wird dann besonders vorteilhaft durch eine Regelung minimiert, indem als die Vorzugsrichtung für das Richtungshören die Hörrichtung eingestellt wird. Dem liegt die Überlegung zugrunde, dass Hören in der vom Anwender gewollten und aktiv angestrebten Hörrichtung gezielt zu erleichtern.

Die Richtcharakteristik ergibt sich beim Hörgerät insbesondere aus einer bestimmten Verarbeitung von Mikrofonsignalen des Hörgeräts. Mit anderen Worten: das Hörgerät weist insbesondere eine Anzahl an Mikrofonen auf, welche im Betrieb jeweils ein Mikrofonsignal erzeugen und die Mikrofonsignale werden mittels einer Steuereinheit derart modifiziert, insbesondere miteinander kombiniert, dass Schallsignale aus der Vorzugsrichtung gegenüber anderen Schallsignalen verstärkt sind. Vorliegend wird nun die Verarbeitung der Mikrofonsignale in Abhängigkeit der Hörrichtung vorgenommen.

Beim Richtungshören wird insbesondere eine Richtkeule gebildet, welche einen Richtwinkel und eine Breite aufweist, wobei der Richtwinkel und die Breite im Detail insbesondere von der Verarbeitung der Mikrofonsignale abhängt. Der Richtwinkel, also die Ausrichtung der Richtkeule, und die Breite sind im Rahmen des Richtungshörbetriebs anpassbar, sodass auch der Richtungshörbetrieb insofern anpassbar ist und dann angepasst wird, indem der Richtwinkel oder die Breite oder beides in Abhängigkeit des Hörvektors angepasst werden. Speziell wird zweckmäßigerweise der Richtwinkel derart eingestellt, dass die Richtkeule in Hörrichtung zeigt.

Die Bestimmung der Hörrichtung ist aber nicht ausschließlich bei Ausrichtung der Richtkeule vorteilhaft, sondern auch, um zu bestimmen, ob überhaupt ein Richtungshören gewollt ist, ob also überhaupt der Richtungshörbetrieb eingestellt werden soll. In einer vorteilhaften Ausgestaltung ist dann der Betriebsmodus ein omnidirektionaler Hörbetrieb, welcher eingestellt wird, falls die Ermittlung der Hörrichtung fehlschlägt. Falls der Anwender nicht in einer bestimmten Richtung hören will, schlägt die Bestimmung der Hörrichtung zwangsläufig fehl und es kann keine Hörrichtung ermittelt werden. Insofern wird hier der Willen des Anwenders nach einem omnidirektionalen, also unfokussierten Hören in allen Richtungen erkannt. In diesem Fall wird dann der omnidirektionale Hörbetrieb eingestellt, bei welchem keine Vorzugsrichtung eingestellt ist, sondern bei welchem insbesondere Schallsignale aus allen Richtungen gleichermaßen an den Anwender ausgegeben werden.

Denkbar ist auch, dass der Anwender möglicherweise zwar nicht den omnidirektionalen Hörbetrieb wünscht, aber aufgrund anderer Umstände oder Fehler die Ermittlung der Hörrichtung fehlschlägt, z.B. bei einer Fehlpositionierung des Hörgeräts derart, dass die Messung des neuronalen Signals fehlerhaft ist. Auch hier ist dann z.B. aus Sicherheitsgründen der omnidirektionale Hörbetrieb vorteilhaft.

In einer geeigneten Ausgestaltung wird der Hörvektor anhand der Hörrichtung einer von fünf Richtungsklassen zugeordnet, nämlich "vorne", "hinten", "links", "rechts" oder "unfokussiert", und jeder dieser Richtungsklassen ein Betriebsmodus zugeordnet ist, nämlich "direktionales Hören nach vorne", "direktionales Hören nach hinten", "direktionales Hören nach links", "direktionales Hören nach rechts" oder "omnidirektionales Hören", und es wird derjenige Betriebsmodus eingestellt wird, welcher der Richtungsklasse zugeordnet ist, welcher der Hörvektor angehört. Die Reduzierung auf die fünf genannten Klassen vereinfacht die Anpassung oder Einstellung des Betriebsmodus erheblich. Zudem wird durch diese Vereinfachung ein entsprechend großer Toleranzbereich für die Ermittlung der Höranstrengung und speziell der Hörrichtung bereitgestellt, sodass selbst bei einer ungenauen Bestimmung der Höranstrengung dennoch zuverlässig ein hinreichend passender Betriebsmodus eingestellt wird.

Bei der Anwendung des Hörvektors bei einem Hörgerät ist bereits ein zweidimensionaler Hörvektor geeignet, d.h. ein Hörvektor, welcher lediglich in einer Ebene liegt. Diese Ebene ist eine Hörebene des Anwenders und erstreckt sich horizontal, sodass die Hörrichtung entsprechend beschränkt ist auf links, rechts, vorne und hinten. In einer vorteilhaften Ausgestaltung ist der Hörvektor jedoch dreidimensional und beschreibt somit eine Hörrichtung in einem Hörraum. Mit einem solchen Hörvektor lassen sich in einer geeigneten Ausgestaltung zusätzlich zu den oben beschriebenen Richtungen links, rechts, vorne, hinten und unfokussiert auch die Richtungen oben und unten abbilden.

In einer besonders bevorzugten Ausgestaltung wird der Hörvektor nicht diskret auf die vorgenannten wenigen Richtungen abgebildet, sondern vielmehr hochaufgelöst ermittelt. Mit anderen Worten: ein dreidimensionaler Raum um den Anwender herum wird in eine Mehrzahl von Richtungen segmentiert, wobei jede Richtung einem Raumwinkel in dem dreidimensionalen Raum entspricht und wobei jeder Richtung ein Betriebsmodus zugeordnet ist, zum Hören in der jeweiligen Richtung, d.h. zur bevorzugten Ausgabe von Schallsignalen aus dem zugehörigen Raumwinkel. Der Hörvektor wird somit über den dreidimensionalen Raum hinweg kontinuierlich dargestellt, sodass eine besonders hohe Richtungsauflösung erzielt wird. Damit wird dann derjenige Betriebsmodus eingestellt, welcher der Richtung zugeordnet ist, welche dem Hörvektor entspricht. Eine Segmentierung des Raums und Darstellung des Hörvektors erfolgt also nicht beschränkt auf die sechs Raumrichtungen links, recht, vorne, hinten, oben und unten, sondern deutlich feiner aufgelöst. Bei der oben beschriebenen groben Auflösung mit den sechs spezifischen Richtungen, wird der dreidimensionale Raum in sechs Raumwinkel aufgeteilt. Bei der hochaufgelösten Segmentierung wird der dreidimensionale Raum dagegen in deutlich mehr als sechs Raumwinkel aufgeteilt, vorzugsweise wenigstens in 100 Raumwinkel. Beim einem Richtungshören insbesondere wie oben beschrieben wird dann die Vorzugsrichtung genau in der Hörrichtung eingestellt und nicht lediglich näherungsweise auf eine begrenzte Anzahl von Richtungsklassen abgebildet.

Die Kenntnis der Hörrichtung ist aber nicht nur bei der Anpassung und Einstellung eines Richtungshörbetriebs vorteilhaft, sondern ganz allgemein auch für eine Anpassung von jeglichen Algorithmen, welche das Hörgerät steuern. Bevorzugterweise wird der Betriebsmodus daher angepasst, indem insbesondere in Abhängigkeit der Hörrichtung eine Rauschreduktion, eine Verstärkung, eine Kompression, ein Audio-Streaming, ein Tinnitus-Algorithmus oder eine Eigenstimmenerkennung angepasst wird. Die vorstehende Liste an Algorithmen ist nicht abschließend, die genannten Algorithmen werden jedoch besonders bevorzugt angepasst. Das Verhalten eines jeweiligen Algorithmus ist durch einen oder mehrere der Betriebsparameter des Hörgeräts bestimmt. Insofern erfolgt durch eine Anpassung der Betriebsparameter typischerweise auch eine Anpassung eines davon abhängigen Algorithmus. Ein jeweiliger Algorithmus dient der Signalverarbeitung insbesondere innerhalb des Hörgeräts und bestimmt, in welcher Weise die Mikrofonsignale modifiziert und schließlich ausgegeben werden. Dabei wird ein jeweiliger Algorithmus vorteilhafterweise derart angepasst, dass die Intensität der Höranstrengung reduziert wird.

Die Verstärkung bestimmt dann insbesondere, in welcher Weise die Mikrofonsignale und allgemein ein Eingangssignal verstärkt wird. Die Verstärkung ist vorzugsweise frequenzabhängig. Die Verstärkung wird vorzugsweise im Rahmen des Richtungshörens angepasst, nämlich derart, dass die Signale aus der Vorzugsrichtung stärker verstärkt werden. Generell wird die Verstärkung zur Reduzierung der Intensität der Höranstrengung zweckmäßigerweise erhöht, vorteilhafterweise allerdings lediglich für Schallsignale aus der Hörrichtung.

Beim Audio-Streaming wird durch einen Audio-Streaming-Algorithmus eine Signalquelle ausgewählt. Beispielsweise weist das Hörgerät als Signalquellen ein Mikrofon oder Mikrofonarray auf, eine Datenübertragungsschnittstelle, eine Telefonspule und eine Audioschnittstelle auf oder eine Teilmenge hiervon. Der Audio-Streaming-Algorithmus wählt nun eine dieser Signalquellen zur Ausgabe an den Anwender aus. In Kenntnis der Hörrichtung ist nun eine verbesserte Auswahl möglich. Beispielsweise hört der Anwender über die Audioschnittstelle Musik, während sich dem Anwender beispielsweise ein Gesprächspartner nähert, welchem der Anwender nunmehr zuhören möchte. Diese Intention wird dann erkannt, indem die Hörrichtung ermittelt wird, welche in Richtung des Gesprächspartners zeigt. Das Audio-Streaming wird dann angepasst, indem das Mikrofon als Signalquelle ausgewählt wird und indem die Audioschnittstelle insbesondere ausgeschaltet wird. Die Anpassung des Audio-Streaming trägt beispielsweise dadurch zur Reduzierung der Intensität der Höranstrengung bei, dass von mehreren Signalquellen diejenige ausgewählt wird, für welche die Intensität minimiert wird.

Der Tinnitus-Algorithmus ist beispielsweise ein Tinnitus-Maskierer oder ein sogenannter Tinnitus-Noiser. Der Tinnitus-Algorithmus modifiziert ein Eingangssignal typischerweise derart, dass in einem Tinnitusfrequenzbereich das Eingangssignal gefiltert, d.h. abgeschwächt wird, oder dass dem Eingangssignal ein zusätzliches Signal, z.B. ein Rauschsignal beigemischt wird.

Zur Reduzierung der Intensität der Höranstrengung wird zweckmäßigerweise auch die Eigenstimmenerkennung angepasst. Die Eigenstimmenerkennung erkennt die eigene Stimme des Anwenders und filtert diese aus dem Eingangssignal heraus. Dadurch sind für den Anwender andere Schallsignale besser zu verstehen.

Bei der Reduzierung der Intensität der Höranstrengung besteht grundsätzlich die Möglichkeit, dass diese nicht vollständig eliminiert wird und ein Rest an Intensität verbleibt, sodass der Anwender sich zum Hören weiterhin anstrengen muss. In einer vorteilhaften Ausgestaltung wird der Betriebsmodus angepasst oder eingestellt und dabei oder danach ein Hinweis an den Anwender ausgegeben, falls die Intensität einen unteren Intensitätsgrenzwert nicht unterschreitet. Dem liegt die Überlegung zugrunde, dass eine Reduzierung auf eine Intensität oberhalb des unteren Intensitätsgrenzwerts weiterhin eine starke Anstrengung erfordert, welche zu einer entsprechenden Ermüdung oder Reizung des Anwenders führen kann. Dem Anwender wird daher ein Hinweis ausgegeben, dass die Einstellung oder Anpassung des Betriebsmodus nicht weiter optimiert werden kann. Der Anwender kann dann entscheiden, wie weiter zu verfahren ist. In einer zweckmäßigen Variante umfasst der Hinweis den Vorschlag, die Umgebung oder die Situation zu wechseln, z.B. in einen ruhigeren Bereich zu wechseln, eine Hintergrundmusik oder einen Fernseher leiser zu stellen oder ein Telefonat zu pausieren. Der Hinweis und speziell auch der Vorschlag werden insbesondere auch als Beratung oder Counseling bezeichnet. Ein Grundgedanke ist hierbei, dem Anwender Hinweise und Vorschläge zu unterbreiten, welche zu einer weiteren Reduzierung der Intensität der Höranstrengung beitragen, welche aber nicht in der Einflusssphäre des Hörgeräts liegen.

Die Anpassung oder Einstellung des Betriebsmodus anhand der Höranstrengung erfolgt insbesondere automatisch. Zweckmäßigerweise ist die gewählte Anpassung oder Einstellung jedoch verhinderbar und wird dann auch verhindert und sozusagen überschrieben. Die beschriebene intentionsbedingte, d.h. intentionsabhängige Anpassung oder Einstellung des Betriebsmodus wird also übergangen. Dem liegt die Überlegung zugrunde, dass es in bestimmten Situationen vorteilhafter ist, von der Hörintention des Anwenders abzuweichen und stattdessen eine abweichende Anpassung oder Einstellung vorzunehmen.

In einer geeigneten Ausgestaltung ist die Anpassung oder Einstellung des Betriebsmodus durch eine manuelle Eingabe des Anwenders verhinderbar wird durch eine solche manuelle Eingabe auch verhindert. Damit wird dem Anwender ermöglicht, das Ergebnis der automatischen Erkennung der Höranstrengung und der Anpassung oder der Einstellung in Abhängigkeit hiervon zu überschreiben.

Alternativ oder zusätzlich wird die Anpassung oder Einstellung des Betriebsmodus verhindert, falls eine bestimmte Schlüsselsituation vorliegt, welche sich durch einen Schlüsselreiz auszeichnet, auf welchen die Hörintention des Anwenders zunächst nicht gerichtet ist, welcher aber für den Anwender von Bedeutung ist und daher trotz mangelnder Hörintention an den Anwender ausgegeben werden soll. Bei Vorliegen einer Schlüsselsituation wird demnach ungeachtet der Höranstrengung, insbesondere der Hörrichtung, und insbesondere ungeachtet der Hörintention der Schlüsselreiz an den Anwender ausgegeben und dadurch eine versehentliche Unterdrückung im Rahmen der regulären Anpassung oder Einstellung verhindert. Dadurch werden wichtige Schallsignale ungehindert an den Anwender weitergegeben. Schlüsselreize sind z.B. das Geräusch eines herannahenden Fahrzeugs oder Warnsignale aus der Umgebung wie beispielsweise Sirenen oder auch Durchsagen. Solche Schlüsselreize sind für den Anwender ungeachtet von dessen augenblicklicher Hörintention relevant und sollen möglichst nicht unterdrückt werden. Entsprechende Schlüsselsituationen sind dann beispielsweise das Überqueren einer Straße, eine Notfallsituation oder das Warten an einem Bahnsteig. Die Schlüsselsituation oder der Schlüsselreiz oder beide werden beispielsweise durch einen Zusatzsensor oder mittels einer Mikrofonanalyse erkannt.

In einer bevorzugten Ausgestaltung wird die Höranstrengung mittels eines EEG ermittelt, bei welchem das neuronale Signal mittels eines Elektrodenarrays gemessen wird. Das Elektrodenarray weist eine Anzahl an Kontakten oder auch Messkontakten auf, welche am Kopf des Anwenders angeordnet sind und welche ein EEG-Signal erzeugen, aus welchem der Hörvektor bestimmt wird. Die Kontakte werden vorliegend jeweils auch als Elektroden bezeichnet. Die neuronalen Signale sind bioelektrische Signale und insbesondere Hirnströme des Anwenders. Die Kontakte des Elektrodenarrays sind jeweils einzelne elektrische Kontakte oder Pole, welche am Kopf des Anwenders angebracht sind, z.B. auf der Kopfhaut. Zwischen je zwei Kontakten wird dann eine Potentialdifferenz gemessen, welche als ein Sensorsignal an eine Auswerteeinheit weitergeleitet wird. Das Elektrodenarray ist also multipolar ausgebildet, um das Sensorsignal als Potentialdifferenz zwischen zwei einzelnen Kontakten an unterschiedlichen Stellen des Kopfes des Anwenders zu messen. Bei mehr als zwei Kontakten werden demnach auch entsprechend mehrere Sensorsignale erzeugt, welche an die Auswerteeinheit weitergeleitet werden.

Die Auswerteeinheit ist in einer Variante ein Teil der Steuereinheit. In einer besonders vorteilhaften Variante ist die Auswerteeinheit eine externe Auswerteeinheit und ein Teil eines externen Geräts, sodass die Auswertung außerhalb des Hörgeräts durchgeführt wird und auf diese Weise Rechenleistung im Hörgerät gespart wird. Das externe Gerät ist z.B. ein Smartphone oder ein Computer des Anwenders oder ein Server.

Die Auswertung erfolgt insbesondere mittels einer mathematischen Aufbereitung des gemessenen neuronalen Signals. In einer geeigneten Ausgestaltung wird hierzu die Phase jedes einzelnen Sensorsignals extrahiert und dann die Verteilung dieser Phasen untersucht, indem ein Schwerpunkt der Verteilung gebildet wird. Dieser Schwerpunkt und allgemein die Anordnung der Phasen (Englisch: phase clustering) in einer bestimmten Situation werden dann zur Auswertung des neuronalen Signals und zur Bestimmung des Hörvektors herangezogen. Vorzugsweise erfolgt die Aufbereitung wie in Bernarding et al. (a.a.O.) im Abschnitt "Data analysis" beschrieben.

Zur Erzeugung des Sensorsignals ist das Elektrodenarray wenigstens bipolar ausgebildet, d.h. weist wenigstens zwei Kontakte auf, zwischen welchen dann eine Potentialdifferenz gemessen wird. Geeignet ist grundsätzlich auch ein Elektrodenarray mit mehr als zwei Kontakten. Für EEG-Messungen in der neuropsychologischen Forschung oder im klinischen Umfeld werden beispielsweise 32 oder sogar 128 Kontakten verwendet, welche über den gesamten Kopf verteilt angeordnet sind. Einer der Kontakte wird dann zweckmäßigerweise als ein Referenzkontakt verwendet, welcher ein Referenzpotential bereitstellt, gegen welches mit den anderen Kontakten jeweils in einer bipolaren Anordnung gemessen wird. Besonders vorteilhaft ist eine Ausgestaltung derart, dass das Elektrodenarray genau einen Messkontakt und einen Referenzkontakt aufweist, also insgesamt lediglich zwei Kontakte, welche beide in das Gehäuse des Hörgeräts integriert sind und somit jeweils besonders nahe des Ohrs des Anwenders angeordnet sind.

In einer geeigneten Ausgestaltung ist das EEG-Signal ein Signalmuster und der Hörvektor wird bestimmt, indem das gemessene Signalmuster mit einer Anzahl an vorbekannten Signalmustern verglichen wird. Das Signalmuster ist insbesondere ein räumliches Signalmuster, welches sich aus der Ausgestaltung des Elektrodenarrays und speziell der Anordnung der Kontakte ergibt. Jeder Kontakt misst an einer bestimmten Position am Kopf, sodass die Messwerte der Kontakte ebenjener Position zugeordnet werden und dadurch die Messwerte räumlich verteilt sind und das Signalmuster bilden. Das Signalmuster ist beispielsweise zusammengesetzt aus den weiter oben beschriebenen Phasen, d.h. das Signalmuster ist eine Matrix, welche als Einträge die Phasen enthält oder allgemein die vorzugsweise aufbereiteten Sensorsignale der Kontakte. Das Signalmuster wird dann mit vorbekannten Signalmustern verglichen, für welche jeweils der Hörvektor bekannt ist. Der Vergleich ist beispielsweise ein einfacher Bildvergleich, bei welchem die Signalmuster als Bilder miteinander verglichen werden. Die vorbekannten Signalverteilungen werden beispielsweise in einem Trainingsverfahren oder in einer Fitting Session bestimmt. Alternativ oder zusätzlich werden die vorbekannten Signalmuster über eine externe Datenbank bezogen.

Vorliegend ist das Elektrodenarray möglichst kompakt ausgebildet. Das Elektrodenarray ist dazu in einer geeigneten Ausgestaltung lediglich im Bereich des Ohrs des Anwenders, genauer gesagt im Bereich der Ohrmuschel angeordnet. Mit anderen Worten: das Elektrodenarray ist vorzugsweise höchstens 5cm vom Ohr, besonders bevorzugt höchstens 2cm entfernt davon angeordnet. Dadurch ist gewährleistet, dass das Elektrodenarray lediglich über einen kleinen Teil des Kopfes des Anwenders verteilt ist und dadurch besonders alltagstauglich ist.

In einer bevorzugten Ausgestaltung weist das Elektrodenarray lediglich höchstens fünf, besonders bevorzugt genau zwei Kontakte auf. Auch eine solche Beschränkung der Kontaktanzahl trägt zur Kompaktheit des Elektrodenarray und zur Alltagstauglichkeit des Hörgeräts und des Verfahrens bei. Der Reduzierung der Kontaktanzahl vor allem im Vergleich zum klinischen Umfeld liegt insbesondere die Beobachtung zugrunde, dass für eine hinreichend genaue Bestimmung des Hörvektors eine eingeschränkte Anzahl an Kontakten völlig ausreichend ist. Besonders im Zusammenhang mit der oben beschriebene Klassifizierung des Hörvektors in wenige und insbesondere lediglich fünf Klassen ist eine vorteilhafte Reduzierung der Kontaktanzahl und eine vereinfachte Messung des neuronalen Signals möglich und wird daher vorteilhafterweise auch durchgeführt. Da die Anforderungen an die Genauigkeit aufgrund der lediglich groben Klassifizierung entsprechend gering sind, muss auch das neuronale Signal nicht besonders genau gemessen werden, sodass dann zweckmäßigerweise ein entsprechend kompaktes Elektrodenarray verwendet wird, welches lediglich wenige Kontakte aufweist.

Die Kontakte des Elektrodenarrays sind vorzugsweise jeweils als Außenkontakte ausgebildet, d.h. als Kontakte, welche außerhalb des Kopfs des Anwenders angeordnet sind. Alternativ ist auch eine Ausgestaltung einer oder mehrerer der Kontakte als Implantat geeignet. Ein jeweiliger Kontakt ist wie oben bereits angedeutet vorzugsweise in ein Gehäuse des Hörgeräts integriert. Dies ermöglicht vor allem auch eine EEG-Messung nicht lediglich im Rahmen einer Fitting Session beim Audiologen unter Verwendung von aufwändiger Apparatur, sondern vielmehr unbemerkt und unsichtbar im Alltag, d.h. im Normalbetrieb des Hörgeräts. Geeignet ist jedoch auch eine Ausgestaltung als separates Elektrodenarray, welches dann über eine Signalleitung oder drahtlos insbesondere an das Hörgerät angebunden ist.

Vorzugsweise wird das neuronale Signal am auditorischen Cortex des Anwenders gemessen, d.h. insbesondere zumindest in der Nähe des auditorischen Cortex. Besonders geeignet ist eine Messung des neuronalen Signals am Mastoid, d.h. am Warzenteil des Schläfenbeins, da der auditorische Cortex in direkter Nähe des Mastoids liegt und gleichzeitig der zugehörige Kontakt nahe am Ohr angebracht wird und somit weitgehend unbemerkt getragen werden kann. Untersuchungen haben gezeigt, dass bereits mit einem einzelnen Kontakt in der Nähe des auditorischen Cortex die Hörrichtung zumindest grob bestimmbar ist. Mehrere Kontakte führen jedoch zu genaueren Ergebnissen.

Das neuronale Signal lässt sich grundsätzlich lediglich einseitig messen und in dieser Form auch zur Bestimmung des Hörvektors nutzen. Bevorzugt ist jedoch eine Ausgestaltung, bei welcher das neuronale Signal beidseitig am Kopf des Anwenders gemessen wird, insbesondere wie oben beschriebene, jeweils in der Nähe des auditorischen Cortex. Dabei werden ein rechter Messwert und ein linker Messwert erzeugt und dann die Hörrichtung durch einen Vergleich des rechten Messwerts und des linken Messwerts bestimmt. In einer ersten Variante werden dazu zumindest zwei Potentialdifferenzen gemessen, indem die beiden Kontakte jeweils gegen einen Referenzkontakt gemessen werden oder gegen einen einzelnen, gemeinsamen Referenzkontakt. Die beiden Messwerte ergeben sich dann jeweils als Potentialdifferenz bezüglich eines Referenzkontakts. In einer anderen Variante ergeben sich die beiden Messwerte jeweils als Signal an einem der Kontakte und die Messwerte werden direkt miteinander verglichen, sodass auf einen separaten Referenzkontakt verzichtet wird. Beispielsweise wird die Differenz oder das Verhältnis der beiden Messwerte gebildet.

In einer besonders bevorzugten Ausgestaltung wird ähnlich der vorgenannten Methode mit linkem und rechten Messwert die Intensität der Höranstrengung auf beiden Seiten des Kopfs des Anwenders bestimmt, sodass eine linke Intensität und eine rechte Intensität gemessen werden und die Hörrichtung wird aus den Intensitäten bestimmt. Dem liegt die Erkenntnis zugrunde, dass die Intensität der Höranstrengung je nach Position einer Schallquelle auf beiden Seiten des Kopfes unterschiedlich ist und daher durch eine beidseitige Messung eines jeweiligen neuronalen Signals die Hörrichtung durch Vergleich der linken und der rechten Intensität ermittelt werden kann und daher vorzugsweise auch ermittelt wird. Allgemein wird demnach durch eine Bestimmung der Intensität an unterschiedlichen Stellen des Kopfes eine Lateralisierung ermittelt, d.h. eine räumliche Abhängigkeit der Intensität, aus welcher die Hörrichtung abgeleitet wird. Zweckmäßigerweise erfolgt die beidseitige Messung bei einem binauralen Hörgerät, bei welchem jedes der Einzelgeräte zunächst insbesondere unabhängig vom anderen Einzelgerät die Intensität der Höranstrengung auf einer Seite misst. Die beiden Intensitäten werden dann in einer Steuereinheit in einem der Einzelgeräte oder auf einem externen Gerät zusammengeführt und verglichen, z.B. wird die Differenz oder das Verhältnis der beiden Intensitäten gebildet.

In einer besonders bevorzugten Ausgestaltung ist das Hörgerät ein binaurales Hörgerät und weist zwei Einzelgeräte auf, zum Tragen auf unterschiedlichen Seiten des Kopfes des Anwenders. Grundsätzlich geeignet ist aber auch ein monoaurales Hörgerät, mit lediglich einem Einzelgerät, zur Versorgung lediglich eines Ohrs des Anwenders. Zweckmäßigerweise weist jedes der Einzelgeräte dann ein entsprechendes Elektrodenarray auf, zur Messung des neuronalen Signals auf der jeweiligen Seite. Alternativ zur separaten Messung mittels zweier Elektrodenarrays verwenden beiden Einzelgeräte ein einzelnes Elektrodenarray gemeinsam. Der Hörvektor wird zweckmäßigerweise bestimmt, indem die neuronalen Signale beider Seiten gemeinsam ausgewertet werden. Geeignet ist aber grundsätzlich auch eine Ausgestaltung, bei welcher der Hörvektor durch beide Einzelgeräte zunächst redundant ermittelt wird und dann die beiden Hörvektoren miteinander verglichen oder gemittelt werden, um einen einzelnen Hörvektor zu bestimmen.

In einer zweckmäßigen Ausgestaltung wird der Betriebsmodus aufgrund einer Auswertung eines Sensorsignals eines Zusatzsensors angepasst oder eingestellt, wobei das Sensorsignal klassifiziert wird, indem die Hörrichtung als ein zusätzliches Merkmal verwendet wird. Dem liegt die Überlegung zugrunde, dass auch die Auswertung diverser Zusatzsensoren des Hörgeräts von der zusätzlichen Kenntnis des Hörvektors und speziell der Hörrichtung profitiert. Üblicherweise werden Sensorsignale von einer Steuereinheit klassifiziert, um aus dem Sensorsignal Informationen bezüglich der Umgebung oder der aktuellen Situation zu erhalten. Die Klassifizierung erfolgt anhand von Merkmalen, welche in dem Sensorsignal gesucht werden. Dabei können auch mehrere Sensorsignale mehrerer Zusatzsensoren gemeinsam entsprechend untersucht werden. Das Ergebnis und dessen Korrektheit sind maßgeblich von der Erkennbarkeit und Unterscheidbarkeit der Merkmale abhängig, um eine möglichst fehlerfreie Klassifizierung durchzuführen. Der Hörvektor stellt nun zusätzliche Merkmale bereit, nämlich die Intensität der Höranstrengung und die Hörrichtung, welche in Kombination mit dem Sensorsignal eine genauere Klassifizierung ermöglichen. Der Zusatzsensor ist beispielsweise ein Mikrofon oder ein Beschleunigungssensor oder Ähnliches.

Vorzugsweise wird die Einstellung oder Anpassung des Betriebsmodus im Rahmen einer Optimierung durchgeführt und Betriebsmodus, welcher auf diese Weise zur Reduzierung der Intensität der Höranstrengung angepasst oder eingestellt wurde wird als optimierter Betriebsmodus gespeichert, kurz: die Anpassung oder Einstellung wird gespeichert. Der optimierte Betriebsmodus wird dann automatisch wieder eingestellt, ausgewählt oder hergestellt, wenn dieselbe oder zumindest eine ähnliche Situation erneut auftritt, wenn also insbesondere derselbe Hörvektor wieder auftritt, welcher ursprünglich Anlass der Optimierung war. Auf diese Weise wird in bekannten Situationen zunächst auf eine Messung des neuronalen Signals und eine entsprechende Auswertung vorteilhaft verzichtet, es wird vielmehr auf die bereits durchgeführte und gespeicherte Optimierung zurückgegriffen. Dies erfolgt beispielsweise im Rahmen einer Fitting Session oder in einem Trainingsverfahren. In einer geeigneten Variante wird die Optimierung vom Anwender selbst vorgenommen, z.B. mittels eines speziellen Trainingsprogramms.

In einer geeigneten Ausgestaltung wird eine anwenderspezifische Optimierung durchgeführt, d.h. der Betriebsmodus wird anwenderspezifische optimiert. Dabei wird für eine gegebene Hörrichtung ein Betriebsmodus gefunden, welcher für den Anwender die Intensität der Höranstrengung minimiert. Dieser Betriebsmodus ist dann ein hinsichtlich des Anwenders spezifischer und individueller optimierter Betriebsmodus. Dieser Betriebsmodus wird gespeichert und erneut eingestellt, falls dieselbe Hörrichtung erneut ermittelt wird. Insgesamt wird also für insbesondere jede Hörrichtung ein auf den Anwender angepasster, optimierter Betriebsmodus gefunden.

In einer weiteren geeigneten Ausgestaltung wird eine situationsspezifische Optimierung durchgeführt, d.h. der Betriebsmodus wird situationsspezifisch, d.h. insbesondere auch umgebungsspezifisch optimiert. In einer gegebenen Umgebungssituation wird der Hörvektor ermittelt und es wird ein Betriebsmodus bestimmt, welcher in der gegebenen Umgebungssituation die Intensität der Höranstrengung minimiert. Dieser Betriebsmodus wird dann bei einer wiederholten Erkennung der Umgebungssituation erneut eingestellt wird. Im Betrieb des Hörgeräts wird dann zunächst die aktuelle Umgebungssituation, kurz die Situation, bestimmt, z.B. mittels eines Zusatzsensors oder im Rahmen einer Mikrofonanalyse, und dann der bereits für diese Umgebungssituation optimierte Betriebsmodus eingestellt, falls ein solcher bereits ermittelt wurde. Eine Umgebungssituation ist beispielsweise ein Konzert, oder eine Cocktailparty, oder eine Konversation mit einem Gegenüber oder Ähnliches.

Besonders zweckmäßig ist auch eine Kombination der beiden vorgenannten Ausgestaltungen, sodass dann eine anwenderspezifische und zugleich situationsspezifische Optimierung erfolgt und dann bei Wiedererkennen einer bestimmten Hörrichtung in einer bestimmten Situation der hierfür optimierte Betriebsmodus eingestellt.

Vorteilhaft ist zudem auch ein Datenaustausch, bei welchem die Ergebnisse der Optimierung und allgemein die Anpassung oder Einstellung des Betriebsmodus bei einem Anwender auch anderen Anwendern zugänglich gemacht wird. Hierzu wird der angepasste oder eingestellte Betriebsmodus zusammen mit Zusatzinformationen an eine externe Datenbank übermittelt und auf diese Weise anderen Anwendern zur Nutzung zur Verfügung gestellt.

In einer zweckmäßigen Ausgestaltung gehört der Anwender einer Klasse von Anwendern an. Die verschiedenen Anwender sind beispielsweise nach Art der Hörschädigung, Alter, Hörgewohnheiten oder ähnlichen Parametern in Klassen eingeordnet. In einer gegebenen Umgebungssituation wird dann ein Betriebsmodus bestimmt, welcher die Intensität der Höranstrengung minimiert, und dieser Betriebsmodus wird in einer externen Datenbank gespeichert, um bei einem anderen Anwender derselben Klasse oder in derselben Umgebungssituation oder beides eingestellt zu werden.

Umgekehrt gehört der Anwender einer Klasse von Anwendern an und in einer geeigneten Ausgestaltung wird in einer gegebenen Umgebungssituation das Hörgerät eingestellt, indem aus einer externen Datenbank ein Betriebsmodus für ebendiese Klasse von Anwendern oder für ebendiese Umgebungssituation entnommen wird oder beides und als ein Ausgangspunkt zur Minimierung der Intensität verwendet wird. Das Hörgerät ruft demnach von der externen Datenbank die Optimierungsergebnisse für ähnliche Anwender oder für ähnliche Umgebungssituationen oder beides ab und schlägt diese dem Anwender zur Verwendung vor. Die Optimierungsergebnisse dienen dann auch als Ausgangspunkte für eine weitere, insbesondere anwenderspezifische Optimierung. In diesem Zusammenhang ist auch ein Bewertungssystem vorteilhaft, bei welchem der Anwender einen von der externen Datenbank heruntergeladenen Betriebsmodus bewertet, sodass zukünftig anderen Anwendern z.B. aufgrund deren Klasse ein besser bewerteter Betriebsmodus eher vorgeschlagen wird.

Das Hörgerät ist vorzugsweise ein sogenanntes BTE-Gerät, welches hinter dem Ohr getragen wird. Dies schließt sogenannte RIC-Geräte ein, bei welchen der Hörer in den Gehörgang eingesetzt ist, das übrige Hörgerät jedoch außerhalb davon getragen wird. Andere Bauformen wie z.B. ITO (in dem Ohr) oder CIC (vollständig im Gehörgang) sind jedoch grundsätzlich auch geeignet. Die Erfindung ist jedoch nicht auf ein Hörgerät zur Versorgung einer hörgeschädigten Person beschränkt. In einer ebenfalls geeigneten Ausgestaltung ist das Hörgerät ein Kopfhörer oder ein ähnliches Gerät zur Schallausgabe. Wesentlich ist, dass das Hörgerät einen Hörer zur Schallausgabe aufweist.

Die Höranstrengung wird bevorzugterweise fortlaufend in einem Normalbetrieb des Hörgeräts ermittelt wird, d.h. insbesondere im Alltag des Anwenders, während dieser das Hörgerät trägt und normal nutzt. Der Betriebsmodus wird ebenso fortlaufend in Abhängigkeit des Hörvektors angepasst oder eingestellt. Hierbei wird insbesondere das neuronale Signal fortlaufend gemessen und dadurch sozusagen überwacht, sodass sofort auf Änderungen reagiert wird. Insbesondere wird dabei wie weiter oben bereits beschrieben die fortlaufende oder oszillierende Aktivität, insbesondere EEG-Aktivität des Anwenders gemessen und gerade keine lediglich reflexartige oder ereigniskorrelierte Aktivität. Die Höranstrengung wird demnach im Rahmen einer Hintergrundmessung oder Hintergrundüberwachung und vorzugsweise andauernd, d.h. fortlaufend ermittelt und somit überwacht. Das Verfahren dient nicht lediglich einer initialen Einstellung des Hörgeräts beim Audiologen in einer Fitting Session, sondern wird gerade im Normalbetrieb ausgeführt, um fortlaufend und bedarfsgerecht eine Optimierung des Betriebsmodus hinsichtlich der Höranstrengung zu erzielen.

Nachfolgend wird ein Ausführungsbeispiel der Erfindung anhand einer Zeichnung näher erläutert. Darin zeigen jeweils schematisch:
- Fig. 1: ein Ohr eines Anwenders und ein Hörgerät,
- Fig. 2: ein Verfahren zum Betrieb des Hörgeräts,
- Fig. 3: mehrere Messergebnisse einer EEG-Messung, und
- Fig. 4: mehrere Auswertungen von Messungen eines neuronalen Signals.

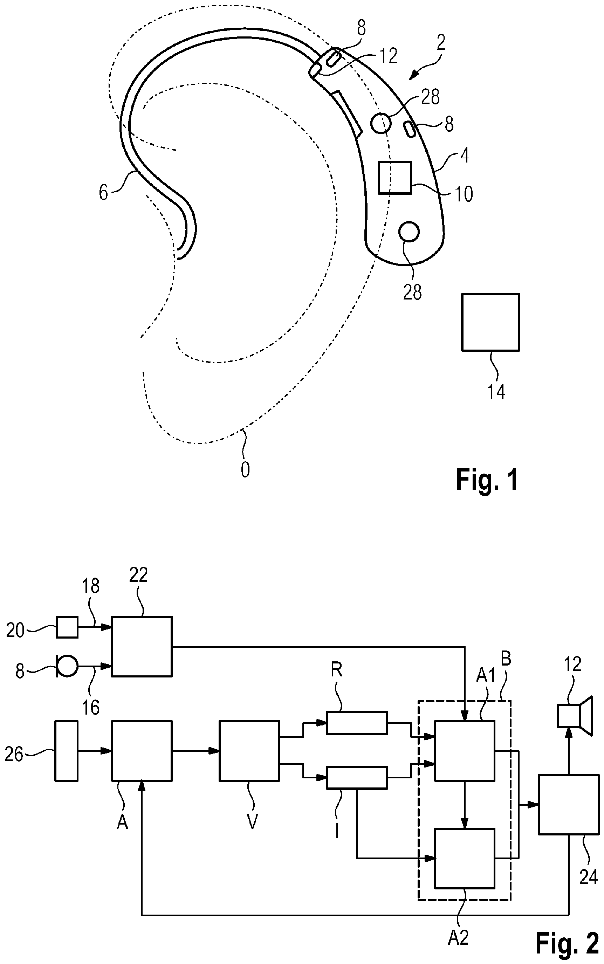

In Fig. 1 ist ein Hörgerät 2 dargestellt, welches hinter einem Ohr O eines nicht weiter dargestellten Anwenders getragen wird. Das Hörgerät 2 ist hier ein BTE-Hörgerät, mit einem Gehäuse 4, welches hinter dem Ohr O getragen wird und von welchem ausgehend sich ein Schallschlauch 6 bis in den Gehörgang erstreckt. Das Hörgerät 2 weist weiterhin eine Anzahl an Mikrofonen 8 auf, welche Schallsignale aus der Umgebung des Anwenders aufnehmen. Diese Schallsignale werden dann mittels einer Steuereinheit 10 modifiziert, insbesondere verstärkt, und dann über einen Hörer 12 ausgegeben. Vom Hörer 12 aus gelangen die modifizierten Schallsignale dann über den Schallschlauch 6 in das Ohr O. Zum sicheren Halt des Schallschlauchs 6 ist endseitig an diesem ein nicht näher gezeigtes Ohrstück angebracht, welches in den Gehörgang eingesetzt wird. Fig. 1 zeigt weiterhin ein externes Gerät 14, welches beispielsweise ein Smartphone oder ein Server ist. Das Hörgerät 2 und das externe Gerät 14 sind zur Datenübertragung untereinander ausgebildet, z.B. mittels Drahtloskommunikation.

In Fig. 1 ist lediglich ein Einzelgerät eines binauralen Hörgeräts 2 gezeigt. Das Hörgerät 2 weist demnach zwei entsprechende Einzelgeräte wie in Fig.1 gezeigt auf, welche dann entsprechend auf unterschiedlichen Seiten des Kopfs des Anwenders getragen werden. In einer Variante ist das Hörgerät jedoch ein monoaurales Hörgerät 2 und weist nur ein Einzelgerät auf.

In Fig. 2 ist gezeigt, wie im Betrieb des Hörgeräts 2 ein neuronales Signal des Anwenders gemessen wird und daraus eine Höranstrengung H des Anwenders ermittelt wird. Aus dem neuronalen Signal wird sowohl eine Intensität I der Höranstrengung H als auch eine Hörrichtung R ermittelt werden, wobei die Intensität I und die Hörrichtung R einen Hörvektor V bilden und wobei ein Betriebsmodus B des Hörgeräts 2 in Abhängigkeit der Hörrichtung R angepasst oder eingestellt wird, zur Reduzierung der Intensität I der Höranstrengung H.

Im Rahmen des Betriebs des Hörgeräts 2 werden zunächst mittels der Mikrofone 8 Schallsignale aus der Umgebung aufgenommen und in Mikrofonsignale 16 umgewandelt. Diese bilden gemeinsam mit Signalen 18 eines oder mehrerer Zusatzsensoren 20 einen Eingang 22. Die Mikrofonsignale 16 werden nun durch Algorithmen A1, A2 modifiziert und an einen Ausgang 24 weitergegeben und mittels des Hörers 12 ausgegeben. Die Modifizierung der Mikrofonsignale 16 ist demnach von den Algorithmen A1, A2 abhängig. Diese bilden zusammen einen Betriebsmodus B. Dieser wird nun im Betrieb in Abhängigkeit des Hörvektors V und speziell der Hörrichtung R angepasst, um die Intensität I der Höranstrengung H möglichst gering zu halten.

In Fig.1 wird die Höranstrengung H mittels eines EEG ermittelt, bei welchem das neuronale Signal mittels eines Elektrodenarrays 26 gemessen wird. Das Elektrodenarray 26 weist eine Anzahl an Kontakten 28 auf, auch als Messkontakte oder Elektroden bezeichnet, welche am Kopf des Anwenders angeordnet sind und welche ein EEG-Signal erzeugen, aus welchem der Hörvektor V bestimmt wird. Vorliegend werden als neuronales Signal die Hirnströme des Anwenders gemessen. Die Kontakte 28 des Elektrodenarrays 26 sind jeweils einzelne elektrische Kontakte oder Pole, welche am Kopf des Anwenders angebracht sind, z.B. auf der Kopfhaut. Die Kontakte 28 sind in Fig.1 in das Gehäuse 4 des Hörgeräts 2 integriert und liegen dann nahe des Ohrs O am Kopf des Anwenders an. Zwischen je zwei Kontakten 28 wird dann eine Potentialdifferenz gemessen, welche als ein Sensorsignal an eine Auswerteeinheit weitergeleitet wird. Diese ist hier entweder ein Teil der Steuereinheit 10 oder des externen Geräts 14.

Die Auswertung erfolgt mittels einer mathematischen Aufbereitung des gemessenen neuronalen Signals. In Fig.3 ist beispielhaft ein Messergebnis gezeigt, welches aus einem EEG mit einer Vielzahl an Kontakten 28 aufgenommen wurde. Die Messergebnisse zeigen jeweils den Kopf des Anwenders von oben betrachtet, die Blickrichtung ist in der Figur durch eine Spitze jeweils am oberen Rand des Signalmusters gekennzeichnet. Aus den einzelnen Sensorsignalen der Kontakte 28 wurde jeweils die Phase extrahiert und dann die die Phasen räumlich in einem Signalmuster 30 dargestellt. In Fig.3 sind vier unterschiedliche Signalmuster 30 gezeigt, welche zu unterschiedlichen Hörvektoren V gehören. Dabei befindet jeweils in einem bestimmten Winkel relativ zur Medianebene des Anwenders eine Schallquelle, welche der Anwender hören möchte, bezüglich welcher also eine Hörintention vorliegt. Den Signalmustern 30 lässt sich somit die vom Anwender intendierte, also gewollte Hörrichtung R entnehmen. Von links nach rechts beträgt der Winkel relativ zur Medianebene -90°, -30°, +30° und +90°. Deutlich erkennbar sind die Unterschiede zwischen den Signalmustern 30. Durch einen Vergleich mit vorbekannten Signalmustern 30 wird dann der Hörvektor V ermittelt.

Ein Signalmuster 30 wie in Fig.3 gezeigt ist aufgrund der hohen Datenmenge zwar gut geeignet zur Bestimmung des Hörvektors H, benötigt jedoch viele Kontakte 28. Im Ausführungsbeispiel der Fig.1 werden dagegen besonders wenige Kontakte 28 verwendet, nämlich lediglich zwei Kontakte 28 pro Einzelgerät. Das Elektrodenarray 26 ist dadurch lediglich über einen kleinen Teil des Kopfes des Anwenders verteilt ist und dadurch besonders alltagstauglich.

In Fig.4 sind vier Messungen gezeigt, bei welchen jeweils beidseitig am Kopf jeweils am Mastoid des Anwenders das neuronale Signal gemessen wurde. Die Messung auf der linken Seite ist jeweils mit L bezeichnet, die Messung auf der rechten Seite jeweils mit R. In vertikaler Richtung ist jeweils die Intensität I der Höranstrengung A aufgetragen. Die vier Messungen unterscheiden sich wie in Fig.3 durch die Position einer Schallquelle relativ zur Medianebene des Anwenders. Von links nach rechts ist die Schallquelle relativ zur Medianebene in einem Winkel von -90°, -30°, +30° und +90° angeordnet. Deutlich erkennbar ist, dass sich aus den Messungen der Intensität I auch die Hörrichtung R ableiten lässt. Die Messungen lassen sich also lateralisieren und einer Hörrichtung R zuordnen. Generell ist aus Fig.4 erkennbar, dass die Intensität I auf der Seite der Schallquelle größer ist als auf der gegenüberliegenden Seite. Deutlich ist auch, dass sich die Hörrichtung R nicht lediglich qualitativ bestimmen lässt, sondern vielmehr auch quantitativ. Weiterhin ist aus Flg.4 auch erkennbar, dass zur Ermittlung der Hörrichtung R bereits eine Messung am Mastoid ausreichend ist, sodass ein kompaktes Elektrodenarray 26 wie in Fig.1 gezeigt bereits für die Bestimmung des Hörvektors V ausreicht.

Die Kenntnis der intendierten Hörrichtung R des Anwenders wird vorliegend zur Anpassung des Betriebsmodus B hinsichtlich einer Richtcharakteristik des Hörgeräts 2 verwendet. Der Betriebsmodus B ist also ein Richtungshörbetrieb, bei welchem Schallsignale aus einer Vorzugsrichtung relativ zu Schallsignalen aus anderen Richtungen verstärkt ausgegeben werden. Der Richtungshörbetrieb wird nun angepasst, indem die Hörrichtung R als die Vorzugsrichtung eingestellt wird. Der Algorithmus A1 ist dann beispielsweise ein Mikrofonalgorithmus, welcher die Mikrofonsignale 16 der Mikrofone 8 derart modifiziert und mischt, dass sich eine bestimmte Richtcharakteristik ergibt. Diese ist durch eine Richtkeule definiert, welche einen Richtwinkel und eine Breite aufweist, wobei der Richtwinkel und die Breite im Detail insbesondere von der Verarbeitung der Mikrofonsignale 16, als vom Algorithmus A1 abhängt. Der Richtwinkel, also die Ausrichtung der Richtkeule, und die Breite werden dann im Rahmen des Richtungshörbetriebs angepasst, indem der Algorithmus A1 angepasst wird und somit der Betriebsmodus B, sodass im Ergebnis der Richtwinkel oder die Breite oder beides in Abhängigkeit des Hörvektors V angepasst werden, genauer gesagt, dass der Richtwinkel derart eingestellt, dass die Richtkeule in Hörrichtung R zeigt.

Die Kenntnis der Hörrichtung R ist aber nicht nur wie oben beschrieben bei der Anpassung und Einstellung eines Richtungshörbetriebs vorteilhaft, sondern ganz allgemein auch für eine Anpassung von jeglichen Algorithmen A1, A2, welche das Hörgerät 2 steuern. Dies ist in Fig.2 durch den weiteren Algorithmus A2 angedeutet, welcher z.B. eine Rauschreduktion, eine Verstärkung, eine Kompression, ein Audio-Streaming, ein Tinnitus-Algorithmus oder eine Eigenstimmenerkennung ist.

### Bezugszeichenliste

- 2: Hörgerät
- 4: Gehäuse
- 6: Schallschlauch
- 8: Mikrofon
- 10: Steuereinheit
- 12: Hörer
- 14: externes Gerät
- 16: Mikrofonsignal
- 18: Signal
- 20: Zusatzsensor
- 22: Eingang
- 24: Ausgang
- 26: Elektrodenarray
- 28: Kontakt
- 30: Signalmuster

- A1, A2: Algorithmus
- B: Betriebsmodus
- H: Höranstrengung
- I: Intensität der Höranstrengung
- O: Ohr
- R: Hörrichtung
- V: Hörvektor

## Patentansprüche

1. Verfahren zum Betrieb eines Hörgeräts (2) für einen Anwender,
- wobei ein neuronales Signal des Anwenders gemessen wird und daraus eine Höranstrengung (H) des Anwenders ermittelt wird,
**dadurch gekennzeichnet,**
- **dass** ausschließlich aus dem neuronalen Signal sowohl eine Intensität (I) der Höranstrengung (H) als auch eine Hörrichtung (R) ermittelt werden,
- **dass** die Intensität (I) und die Hörrichtung (R) einen Hörvektor (V) bilden,
- **dass** ein Betriebsmodus (B) des Hörgeräts (2) in Abhängigkeit der Hörrichtung (R) angepasst oder eingestellt wird, zur Reduzierung der Intensität (I) der Höranstrengung (H).

2. Verfahren nach dem vorhergehenden Anspruch,
**dadurch gekennzeichnet,**
**dass** der Betriebsmodus (B) angepasst wird, indem eine Anzahl an Betriebsparametern des Hörgeräts (2) mittels einer Regelung angepasst werden, wobei die Betriebsparameter als Stellgrößen verwendet werden und wobei die Intensität der Höranstrengung als Regelgröße verwendet wird.

3. Verfahren nach einem der vorhergehenden Ansprüche,
**dadurch gekennzeichnet,**
**dass** der Betriebsmodus (B) ein Richtungshörbetrieb ist, bei welchem Schallsignale aus einer Vorzugsrichtung relativ zu Schallsignalen aus anderen Richtungen verstärkt ausgegeben werden, und
**dass** der Richtungshörbetrieb angepasst wird, indem die Hörrichtung (R) als die Vorzugsrichtung eingestellt wird.

4. Verfahren nach einem der vorhergehenden Ansprüche,
**dadurch gekennzeichnet,**
**dass** der Betriebsmodus (B) ein omnidirektionaler Hörbetrieb ist, welcher eingestellt wird, falls die Ermittlung der Hörrichtung (R) fehlschlägt.

5. Verfahren nach einem der vorhergehenden Ansprüche,
**dadurch gekennzeichnet,**
**dass** der Hörvektor (V) anhand der Hörrichtung (R) einer von fünf Richtungsklassen zugeordnet wird, nämlich "vorne", "hinten", "links", "rechts" oder "unfokussiert",
**dass** jeder dieser Richtungsklassen ein Betriebsmodus (B) zugeordnet ist, nämlich "direktionales Hören nach vorne", "direktionales Hören nach hinten", "direktionales Hören nach links", "direktionales Hören nach rechts" oder "omnidirektionales Hören", und
**dass** derjenige Betriebsmodus (B) eingestellt wird, welcher der Richtungsklasse zugeordnet ist, welcher der Hörvektor (V) angehört,
und/oder
**dass** der dreidimensionaler Raum um den Anwender herum in eine Mehrzahl von Richtungen segmentiert wird, wobei jede Richtung einem Raumwinkel in dem dreidimensionalen Raum entspricht und wobei jeder Richtung ein Betriebsmodus (B) zugeordnet ist, zum Hören in der jeweiligen Richtung,
**dass** der Hörvektor über den dreidimensionalen Raum hinweg kontinuierlich dargestellt wird,
**dass** derjenige Betriebsmodus (B) eingestellt wird, welcher der Richtung zugeordnet ist, welche dem Hörvektor (V) entspricht.

6. Verfahren nach einem der vorhergehenden Ansprüche,
**dadurch gekennzeichnet,**
**dass** der Betriebsmodus (B) angepasst oder eingestellt wird und dass dabei oder danach ein Hinweis an den Anwender ausgegeben wird, falls die Intensität (I) einen unteren Intensitätsgrenzwert nicht unterschreitet.

7. Verfahren nach einem der vorhergehenden Ansprüche,
**dadurch gekennzeichnet,**
**dass** die Anpassung oder Einstellung des Betriebsmodus (B) durch eine manuelle Eingabe des Anwenders verhinderbar ist,
und/oder
**dass** die Anpassung oder Einstellung des Betriebsmodus verhindert wird, falls eine bestimmte Schlüsselsituation vorliegt, welche sich durch einen Schlüsselreiz auszeichnet, welcher ungeachtet der Höranstrengung (H) an den Anwender ausgegeben wird.

8. Verfahren nach einem der vorhergehenden Ansprüche,
**dadurch gekennzeichnet,**
**dass** die Höranstrengung (H) mittels eines EEG ermittelt wird, bei welchem das neuronale Signal mittels eines Elektrodenarrays (26) gemessen wird, welches eine Anzahl an Kontakten (28) aufweist, welche am Kopf des Anwenders angeordnet sind und welche ein EEG-Signal erzeugen, aus welchem der Hörvektor (V) bestimmt wird, wobei bevorzugterweise die Kontakte (28) lediglich in einem Bereich von höchstens 5cm, besonders bevorzugt höchstens 2cm um das Ohr (O) des Anwenders herum angeordnet sind.

9. Verfahren nach dem vorhergehenden Anspruch,
**dadurch gekennzeichnet,**
**dass** das EEG-Signal ein Signalmuster (30) ist und dass der Hörvektor (V) bestimmt wird, indem das gemessene Signalmuster (30) mit einer Anzahl an vorbekannten Signalmustern (30) verglichen wird.

10. Verfahren nach einem der vorhergehenden Ansprüche,
**dadurch gekennzeichnet,**
**dass** das neuronale Signal auf beiden Seiten des Kopfes des Anwenders jeweils am auditorischen Cortex gemessen wird, wobei ein rechter Messwert und ein linker Messwert erzeugt werden, und dass die Hörrichtung (R) durch einen Vergleich des rechten Messwerts und des linken Messwerts bestimmt wird.

11. Verfahren nach einem der vorhergehenden Ansprüche,
**dadurch gekennzeichnet,**
**dass** der Betriebsmodus (B) aufgrund einer Auswertung eines Sensorsignals (18) eines Zusatzsensors (20) angepasst oder eingestellt wird, wobei das Sensorsignal (18) klassifiziert wird, indem die Hörrichtung (R) als ein zusätzliches Merkmal verwendet wird.

12. Verfahren nach einem der vorhergehenden Ansprüche,
**dadurch gekennzeichnet,**
**dass** eine anwenderspezifische Optimierung durchgeführt wird, bei welcher für eine gegebene Hörrichtung (R) ein Betriebsmodus (B) gefunden wird, welcher für den Anwender die Intensität (I) der Höranstrengung (H) minimiert, und dass jener Betriebsmodus (B) gespeichert wird und erneut eingestellt wird, falls dieselbe Hörrichtung (R) erneut ermittelt wird, und/oder
**dass** eine situationsspezifische Optimierung durchgeführt wird, wobei in einer gegebenen Umgebungssituation der Hörvektor (V) ermittelt wird und ein Betriebsmodus (B) bestimmt wird, welcher in dieser Umgebungssituation die Intensität (I) der Höranstrengung (H) minimiert, und wobei bei einer wiederholten Erkennung der Umgebungssituation jener Betriebsmodus (B) erneut eingestellt wird.

13. Verfahren nach einem der vorhergehenden Ansprüche,
**dadurch gekennzeichnet,**
**dass** der Anwender einer Klasse von Anwendern angehört und dass in einer gegebenen Umgebungssituation ein Betriebsmodus (B) bestimmt wird, welcher die Intensität (I) der Höranstrengung (H) minimiert, und dass dieser Betriebsmodus (B) in einer externen Datenbank gespeichert wird, um bei einem anderen Anwender derselben Klasse oder in derselben Umgebungssituation oder beides eingestellt zu werden,
und/oder
**dass** der Anwender einer Klasse von Anwendern angehört und dass in einer gegebenen Umgebungssituation das Hörgerät (2) eingestellt wird, indem aus einer externen Datenbank ein Betriebsmodus (B) für ebendiese Klasse von Anwendern oder für ebendiese Umgebungssituation entnommen wird oder beides und als ein Ausgangspunkt zur Minimierung der Intensität (I) verwendet wird.

14. Verfahren nach einem der vorhergehenden Ansprüche,
**dadurch gekennzeichnet,**
**dass** die Höranstrengung (H) fortlaufend in einem Normalbetrieb des Hörgeräts (2) ermittelt wird und dass der Betriebsmodus (B) fortlaufend in Abhängigkeit des Hörvektors (V) angepasst oder eingestellt wird.

15. Hörgerät (2), welches eine Steuereinheit (10) aufweist, welche derart ausgebildet ist, dass ein Verfahren nach einem der vorhergehenden Ansprüche ausgeführt wird.

## Claims

1. Method for operating a hearing aid (2) for a user,
- wherein a neuronal signal of the user is measured and a listening effort (H) of the user is established therefrom, **characterized**
- **in that** both an intensity (I) of the listening effort (H) and a hearing direction (R) are established exclusively from the neuronal signal,
- **in that** the intensity (I) and the hearing direction (R) form a hearing vector (V),
- **in that** a mode of operation (B) of the hearing aid (2) is adapted or set depending on the hearing direction (R), for the purposes of reducing the intensity (I) of the listening effort (H).

2. Method according to the preceding claim,
**characterized**
**in that** the mode of operation (B) is adapted by virtue of adapting a number of operating parameters of the hearing aid (2) by means of a closed-loop control, wherein the operating parameters are used as manipulated variables and wherein the intensity of the listening effort is used as a controlled variable.

3. Method according to either of the preceding claims,
**characterized**
**in that** the mode of operation (B) is a directional hearing operation, in which sound signals from a preferred direction are output in amplified fashion relative to sound signals from other directions, and
**in that** the directional hearing operation is adapted by virtue of setting the hearing direction (R) as the preferred direction.

4. Method according to any one of the preceding claims,
**characterized**
**in that** the mode of operation (B) is an omnidirectional hearing operation, which is set if establishing the hearing direction (R) fails.

5. Method according to any one of the preceding claims,
**characterized**
**in that** the hearing vector (V) is assigned on the basis of the hearing directionAL (R) to one of five directional classes, namely "front", "back", "left", "right" or "unfocused",
**in that** each of these direction classes has assigned to it a mode of operation (B), namely "directional hearing to the front", "directional hearing to the back", "directional hearing to the left", "directional hearing to the right" or "omnidirectional hearing", and
**in that** the mode of operation (B) which is assigned to the directional class to which the hearing vector (V) belongs is set,
and/or
**in that** the three-dimensional space around the user is segmented into a plurality of directions, wherein each direction corresponds to a solid angle in the three-dimensional space and wherein each direction has assigned to it a mode of operation (B) for hearing in the respective direction,
**in that** the hearing vector is represented continuously over the three-dimensional space,
**in that** the mode of operation (B) which is assigned to the direction corresponding to the hearing vector (V) is set.

6. Method according to any one of the preceding claims,
**characterized**
**in that** the mode of operation (B) is adapted or set and in that, in the process or thereafter, a notification is output to the user should the intensity (I) not drop below a lower intensity limit value.

7. Method according to any one of the preceding claims,
**characterized**
**in that** adapting or setting of the mode of operation (B) is preventable by manual input by the user,
and/or
**in that** adapting or setting of the mode of operation is prevented should a certain key situation be present, said key situation being distinguished by a key stimulus that is output to the user irrespective of the listening effort (H).

8. Method according to any one of the preceding claims,
**characterized**
**in that** the listening effort (H) is established by means of an EEG, in which the neuronal signal is measured by means of an electrode array (26), said electrode array having a number of contacts (28) that are arranged at the head of the user and that produce an EEG signal, from which the hearing vector (V) is determined, wherein the contacts (28) are preferably arranged only in a region of at most 5 cm, particularly preferably at most 2 cm, around the ear (O) of the user.

9. Method according to the preceding claim,
**characterized**
**in that** the EEG signal is a signal pattern (30) and in that the hearing vector (V) is determined by virtue of the measured signal pattern (30) being compared to a number of signal patterns (30) known in advance.

10. Method according to any one of the preceding claims,
**characterized**
**in that** the neuronal signal is measured on both sides of the head of the user, in each case at the auditory cortex, wherein a right measurement value and a left measurement value are produced, and in that the hearing direction (R) is determined by comparing the right measurement value and the left measurement value.

11. Method according to any one of the preceding claims,
**characterized**
**in that** the mode of operation (B) is adapted or set on the basis of an evaluation of a sensor signal (18) of an additional sensor (20), wherein the sensor signal (18) is classified by virtue of the hearing direction (R) being used as an additional feature.

12. Method according to any one of the preceding claims,
**characterized**
**in that** a user-specific optimization is carried out, in which a mode of operation (B) is found for a given hearing direction (R), said mode of operation minimizing the intensity (I) of the listening effort (H) for the user, and in that this mode of operation (B) is stored and set again should the same hearing direction (R) be established again,
and/or
**in that** a situation-specific optimization is carried out, wherein the hearing vector (V) is established in a given ambient situation and a mode of operation (B) is determined, said mode of operation minimizing the intensity (I) of the listening effort (H) in this ambient situation, and wherein this mode of operation (B) is set again in the case of a repeated identification of the ambient situation.

13. Method according to any one of the preceding claims,
**characterized**
**in that** the user belongs to a class of users and in that a mode of operation (B) is determined in a given ambient situation, said mode of operation minimizing the intensity (I) of the listening effort (H), and in that this mode of operation (B) is stored in an external database in order to be set in the case of another user of the same class or in the same ambient situation or in the case of both,
and/or
**in that** the user belongs to a class of users and in that the hearing aid (2) is set in a given ambient situation by virtue of a mode of operation (B) for precisely this class of users or for precisely this ambient situation or for both being taken from an external database and being used as a starting point for minimizing the intensity (I).

14. Method according to any one of the preceding claims,
**characterized**
**in that** the listening effort (H) is established continuously during normal operation of the hearing aid (2) and in that the mode of operation (B) is continuously adapted or set depending on the hearing vector (V).

15. Hearing aid (2) having a control unit (10) which is embodied in such a way that a method according to any one of the preceding claims is carried out.

## Revendications

1. Procédé d'utilisation d'un appareil auditif (2) destiné à un utilisateur,
- dans lequel un signal neuronal de l'utilisateur est mesuré et un effort d'audition (H) de l'utilisateur est déterminé à partir de celui-ci, **caractérisé**
- **en ce qu'**une intensité (I) de l'effort d'audition (H) ainsi qu'une direction d'audition (R) sont déterminées exclusivement à partir du signal neuronal,
- **en ce que** l'intensité (I) et la direction d'audition (R) forment un vecteur d'audition (V),
- **en ce qu'**un mode de fonctionnement (B) de l'appareil auditif (2) est adapté ou réglé en fonction de la direction d'audition (R) afin de réduire l'intensité (I) de l'effort d'audition (H).

2. Procédé selon la revendication précédente, **caractérisé**
**en ce que** le mode de fonctionnement (B) est adapté en adaptant un certain nombre de paramètres de fonctionnement de l'appareil auditif (2) au moyen d'un dispositif de commande, dans lequel les paramètres de fonctionnement sont utilisés en tant que variables de réglage et dans lequel l'intensité de l'effort d'audition est utilisée en tant que grandeur de régulation.

3. Procédé selon l'une des revendications précédentes, **caractérisé**
**en ce que** le mode de fonctionnement (B) est un mode d'audition directionnelle lors duquel des signaux sonores provenant d'une direction préférée sont amplifiés par rapport à des signaux sonores provenant d'autres directions, et
**en ce que** le mode d'audition directionnelle est adapté en réglant la direction d'audition (R) en tant que direction préférée.

4. Procédé selon l'une des revendications précédentes, **caractérisé**
**en ce que** le mode de fonctionnement (B) est un mode d'audition omnidirectionnelle qui est réglé en cas d'échec de la détermination de la direction d'audition (R).

5. Procédé selon l'une des revendications précédentes, **caractérisé**
**en ce que** le vecteur d'audition (V) est associé, sur la base de la direction d'audition (R), à l'une de cinq classes de direction, à savoir « avant », « arrière », « gauche », « droite » ou « non focalisé »,
**en ce qu'**un mode de fonctionnement (B) est associé à chacune desdites classes de direction, à savoir "audition directionnelle avant", "audition directionnelle arrière", "audition directionnelle gauche", "audition directionnelle droite" ou "audition omnidirectionnelle", et
**en ce que** le mode de fonctionnement (B) qui est associé à la classe de direction à laquelle appartient le vecteur d'audition (V) est réglé, et/ou
**en ce que** l'espace tridimensionnel entourant l'utilisateur est segmenté en une pluralité de directions, dans lequel chaque direction correspond à un angle solide dans l'espace tridimensionnel et dans lequel un mode de fonctionnement (B) est associé à chaque direction pour l'audition dans la direction respective,
**en ce que** le vecteur d'audition est représenté en continu dans l'espace tridimensionnel,
**en ce que** le mode de fonctionnement (B) qui est associé à la direction correspondant au vecteur d'audition (V) est réglé.

6. Procédé selon l'une des revendications précédentes, **caractérisé**
**en ce que** le mode de fonctionnement (B) est adapté ou réglé et en ce que, pendant ou après, une indication est délivrée à l'utilisateur dans le cas où l'intensité (I) ne s'abaisse pas en-dessous d'une valeur limite d'intensité inférieure.

7. Procédé selon l'une des revendications précédentes, **caractérisé**
**en ce que** l'adaptation ou le réglage du mode de fonctionnement (B) peut être empêché par une saisie manuelle effectuée par l'utilisateur, et/ou en ce que l'adaptation ou le réglage du mode de fonctionnement est empêché dans le cas où il existe une situation clé déterminée qui se **caractérise par** un stimulus clé qui est délivré à l'utilisateur indépendamment de l'effort d'audition (H).

8. Procédé selon l'une des revendications précédentes, **caractérisé**
**en ce que** l'effort d'audition (H) est déterminé au moyen d'un EEG lors duquel le signal neuronal est mesuré au moyen d'un réseau d'électrodes (26) qui comporte un certain nombre de contacts (28) disposés sur la tête de l'utilisateur et qui génèrent un signal EEG à partir duquel le vecteur d'audition (V) est déterminé, dans lequel les contacts (28) sont de préférence disposés uniquement dans une zone d'au plus 5 cm, de préférence d'au plus 2 cm, autour de l'oreille (O) de l'utilisateur.

9. Procédé selon la revendication précédente, **caractérisé**
**en ce que** le signal EEG est un modèle de signal (30) et en ce que le vecteur d'audition (V) est déterminé en comparant le modèle de signal (30) mesuré à un certain nombre de modèles de signal (30) préalablement connus.

10. Procédé selon l'une des revendications précédentes, **caractérisé**
**en ce que** le signal neuronal est mesuré des deux côtés de la tête de l'utilisateur au niveau du cortex auditif, dans lequel une valeur de mesure droite et une valeur de mesure gauche sont générées, et en ce que la direction d'audition (R) est déterminée en comparant la valeur de mesure droite et la valeur de mesure gauche.

11. Procédé selon l'une des revendications précédentes, **caractérisé**
**en ce que** le mode de fonctionnement (B) est adapté ou réglé sur la base d'une évaluation d'un signal de capteur (18) d'un capteur supplémentaire (20), dans lequel le signal de capteur (18) est classé en utilisant la direction d'audition (R) en tant que caractéristique supplémentaire.

12. Procédé selon l'une des revendications précédentes, **caractérisé**
**en ce qu'**une optimisation spécifique de l'utilisateur est effectuée, lors de laquelle un mode de fonctionnement (B) est trouvé pour une direction d'audition donnée (R), lequel mode de fonctionnement minimise l'intensité (I) de l'effort d'audition (H) pour l'utilisateur, et en ce que ledit mode de fonctionnement (B) est stocké et est à nouveau réglé si la même direction d'audition (R) est à nouveau déterminée, et/ou
**en ce qu'**une optimisation spécifique de la situation est effectuée, dans lequel le vecteur d'audition (V) est déterminé dans une situation ambiante donnée et un mode de fonctionnement (B) est déterminé, lequel minimise l'intensité (I) de l'effort d'audition (H) dans ladite situation ambiante, et dans lequel, lors d'une détection répétée de la situation ambiante, ledit mode de fonctionnement (B) est à nouveau réglé.

13. Procédé selon l'une des revendications précédentes, **caractérisé**
**en ce que** l'utilisateur appartient à une classe d'utilisateurs et en ce que, dans une situation ambiante donnée, un mode de fonctionnement (B) qui minimise l'intensité (I) de l'effort d'audition (H) est déterminé, et en ce que ledit mode de fonctionnement (B) est stocké dans une base de données externe afin d'être réglé chez un autre utilisateur de la même classe ou se trouvant dans la même situation ambiante ou les deux, et/ou
**en ce que** l'utilisateur appartient à une classe d'utilisateurs et en ce que, dans une situation ambiante donnée, l'appareil auditif (2) est réglé en extrayant d'une base de données externe un mode de fonctionnement (B) destiné à ladite classe d'utilisateurs ou à ladite situation ambiante ou aux deux et en l'utilisant comme point de départ de la minimisation de l'intensité (I).

14. Procédé selon l'une des revendications précédentes, **caractérisé**
**en ce que** l'effort d'audition (H) est déterminé en continu lors d'un fonctionnement normal de l'appareil auditif (2) et en ce que le mode de fonctionnement (B) est adapté ou réglé en continu en fonction du vecteur d'audition (V).

15. Appareil auditif (2) comportant une unité de commande (10) conçue pour mettre en œuvre un procédé selon l'une des revendications précédentes.
